# EUROPEAN PATENT APPLICATION

(11) **EP 4 427 784 A1**
(43) Date of publication of application: **11.09.2024**
(21) Application number: 22900478.3
(22) Date of filing: 29.11.2022
(51) Int. Cl.: A61M 16/08, A61M 16/10, A61M 16/00

(54) **AIR INLET STRUCTURE, AIR INLET COMPONENT, NOISE REDUCTION BOX, AND VENTILATION TREATMENT DEVICE**

(30) Priority: 02.12.2021 CN 202111463070
(71) Applicant: BMC (Tianjin) Medical Co., Ltd., Tianjin 301700 (CN)
(72) Inventor: ZHI, Jianxin, Beijing 100041 (CN); ZHUANG, Zhi, Beijing 100041 (CN)
(74) Representative: Grünecker Patent- und Rechtsanwälte PartG mbB
(86) International application number: PCT/CN2022/135089
(87) International publication number: WO 2023/098674

(57) **Abstract**

An air inlet structure (10), an air inlet component, a noise reduction box, and a ventilation treatment device. The air inlet structure (10) comprises one or more air inlet units (11), and each air inlet unit (11) comprises a main air path (111) and at least one branch air path (112). The branch air path (112) is configured to laterally bend and extend from the upstream of the main air path (111) to the downstream of the main air path (111). At the position where the branch air path (112) is in communication with the upstream of the main air path (111), an included angle between the direction of an airflow entering the branch air path (112) and the direction of an airflow in the main air path (111) is an obtuse angle; at the position where the branch air path (112) is in communication with the downstream of the main air path (111), an included angle between the direction of an airflow from the branch air path (112) and the direction of the airflow in the main air path (111) is an acute angle. When the air inlet structure (10) is applied to the ventilation treatment device, noise transmitted from an air inlet channel of a blower (21) can be effectively reduced, and the air inlet structure (10) is convenient to process, simple in structure, and convenient to apply.

## Description

### FIELD

The present disclosure relates to the technical field of air path noise reduction, in particular to an air inlet structure, an air inlet component, a noise reduction box and a ventilation treatment device.

### BACKGROUND

When a respiratory product operates normally, a continuous positive pressure is established by virtue of the high-speed rotation of the blades inside a blower to output air, which is delivered to a patient through a respiratory pipeline and a mask. When the blades of the blower rotate at a high speed, they produce high noise. Though the blower is placed inside the respiratory product, the noise generated by the blower will still be propagated to the spatial environment along an air intake passage, and will affect the patients or other persons in the environment where the product is used. Especially during night time, the noise will seriously affect the sleeping and sleeping quality of every person. Therefore, such a product needs a noise reduction mechanism to reduce the noise generated by the blower, and the noise reduction mechanism is usually disposed on the air intake passage at the front end of the blower.

At present, in respiratory products, a noise reduction mechanism and noise-damping cotton are designed and arranged in the air intake passage at the front end of the blower, to reduce the noise resulted from the high-speed rotation of the blades of the blower.

At present, in respiratory products, the noise reduction solution is usually implemented in the form of a noise reduction mechanism and noise-damping cotton, and the structural form is complex.

### SUMMARY

An object of the present disclosure is to provide an air inlet structure, an air inlet component, a noise reduction box and a ventilation treatment device to solve the above problems.

In order to achieve the above object, in a first aspect, the present disclosure provides an air inlet structure, which comprises one or more air inlet units, each air inlet unit comprises a main air path and at least one branch air path configured to bend laterally and extend from the upstream of the main air path to the downstream of the main air path, wherein at the position where the branch air path is in communication with the upstream of the main air path, an included angle between the direction of an airflow entering the branch air path and the direction of an airflow in the main air path is an obtuse angle; and at the position where the branch air path is in communication with the downstream of the main air path, an included angle between the direction of an airflow from the branch air path and the direction of an airflow in the main air path is an acute angle.

Optionally, the air inlet unit comprises a plurality of branch air paths connected in parallel with each other to the main air path.

Optionally, the air inlet structure comprises a plurality of air inlet units in communication with each other sequentially via the main air paths.

Optionally, the main air path is in a linear shape.

Optionally, the branch air path is in an arc shape or trilateral frame shape.

Optionally, the main air path is in a linear shape, each branch air path is in an arc shape, and the air inlet structure comprises a plurality of air inlet units in communication with each other sequentially via the main air paths, and the main air paths of adjacent air inlet units are arranged at an included angle with respect to each other.

Optionally, the main air path is in a linear shape, each branch air path is in a trilateral frame shape, and the air inlet structure comprises a plurality of air inlet units in communication with each other sequentially via the main air paths, and the plurality of main air paths are arranged in a straight line.

In a second aspect, the present disclosure provides an air inlet component, which comprises the air inlet structure described above.

Optionally, the air inlet component has an air inlet and an air outlet, wherein the air inlet is in communication with an air inlet end of the air inlet structure, and the air outlet is in communication with an air outlet end of the air inlet structure.

Optionally, the air inlet component comprises a plurality of air inlet structures arranged side by side.

In a third aspect, the present disclosure provides a noise reduction box, which comprises a housing and a blower, wherein the housing is provided with a mounting cavity, and an air inlet and an air outlet that are in communication with the mounting cavity; the blower is mounted in the mounting cavity, an air intake port of the blower is in communication with the air inlet, and an air discharge port of the blower is in communication with the air outlet; and a communication path between the air inlet and the air intake port is provided with the air inlet structure described above.

Optionally, the housing comprises an upper housing and a lower housing assembled below the upper housing, the mounting cavity is defined by the upper housing and the lower housing together, and the air inlet structure is arranged on the upper housing.

Optionally, the upper housing comprises a top wall and a peripheral wall extending downward from the periphery of the top wall, the air inlet and the air inlet structure are arranged on the top wall, and the top wall is provided with a communication port for communication between an air outlet end of the air inlet structure and the mounting cavity.

Optionally, the air inlet structure is arranged on a top surface of the top wall in a recessed form, and the housing further comprises an upper cover plate, which covers above the top wall.

In a fourth aspect, the present disclosure provides a ventilation treatment device, which comprises the noise reduction box described above.

With the above technical scheme, when the air inlet structure in the present disclosure is used, the airflow entering through the air inlet end of the air inlet structure can flow to the downstream along the main air path and the branch air path respectively; the airflow in the main air path and the airflow in the branch air path are converged at a position where the branch air path is in communication with the main air path at the downstream of the main air path to form an airflow convergence point, so that the air flow speed is increased after the airflows are converged, and the flow of air is promoted.

The noise that enters reversely through the air outlet end of the air inlet structure can be propagated reversely along the main air path and the branch air path respectively; the noise propagated along the main air path and the noise propagated along the branch air path are converged at a position where the branch air path is in communication with the main air path at the upstream of the main air path to form a noise convergence point; at the noise convergence point, the noise propagation direction in the branch air path is opposite or approximately opposite to the noise propagation direction in the main air path, and the noises cancel each other out, thereby an effect of noise reduction is achieved. When the air inlet structure in the present disclosure is applied to the ventilation treatment device, noise transmitted from an air intake passage of a blower can be effectively reduced, and the air inlet structure is convenient to process, simple in structure, and convenient to apply.

Other features and advantages of the present disclosure will be further detailed in the following embodiments.

### BRIEF DESCRIPTION OF THE DRAWINGS

The accompanying drawings are provided herein to facilitate further understanding on the present disclosure and constitute a part of this specification. They are used in conjunction with the following embodiments to explain the present disclosure, but are not intended to constitute any limitation to the present disclosure. In the figures:
Fig. 1 is a structural schematic diagram of an embodiment of the air inlet structure in the present disclosure;
Fig. 2 is a front view of the air inlet structure in Fig. 1, indicating the air flow direction with a solid arrow;
Fig. 3 is a front view of the air inlet structure in Fig. 1, indicating the noise propagation direction with a dotted arrow;
Fig. 4 is a structural schematic diagram of another embodiment of the air inlet structure in the present disclosure;
Fig. 5 is a front view of the air inlet structure in Fig. 4, indicating the air flow direction with a solid arrow;
Fig. 6 is a front view of the air inlet structure in Fig. 4, indicating the noise propagation direction with a dotted arrow;
Fig. 7 is a perspective view of an embodiment of the noise reduction box in the present disclosure;
Fig. 8 is a sectional perspective view of the noise reduction box in Fig. 7;
Fig. 9 is a front view of the noise reduction box in Fig. 8;
Fig. 10 is a sectional view of the noise reduction box in Fig. 7;
Fig. 11 is an exploded view of the noise reduction box in Fig. 7;
Fig. 12 is a sectional perspective view of the upper housing in Fig. 11;
Fig. 13 is a bottom perspective view of the upper housing in Fig. 11;
Fig. 14 is a perspective view of the lower housing in Fig. 11;
Fig. 15 is a perspective view of the blower in Fig. 11;
Fig. 16 is another perspective view of the blower in Fig. 11;
Fig. 17 is a perspective view of the positioning ring in Fig. 11;
Fig. 18 is another perspective view of the positioning ring in Fig. 11; and
Fig. 19 is a sectional view of the positioning ring in Fig. 18.

### Reference Numbers

10 - air inlet structure, 11 - air inlet unit, 111 - main air path, 112 - branch air path, 20 - noise reduction box, 21 - blower, 211 - air intake port, 212 - air discharge port, 213 - blower leg, 214 - positioning column, 22 - mounting cavity, 23 - upper housing, 231 - air inlet, 232 - air outlet, 233 - top wall, 234 - peripheral wall, 235 - communication port, 236 - mounting cylinder, 24 - lower housing, 241 - positioning cylinder, 25 - top cover plate, 26 - upper seal, 27 - lower seal, 28 - positioning ring, 29 - seal ring, 30 - shock-absorbing leg.

### DETAILED DESCRIPTION

Some embodiments of the present disclosure will be detailed below with reference to the accompanying drawings. It may be understood that the embodiments described herein are only provided to describe and explain the present disclosure, but are not intended to constitute any limitation on the present disclosure.

In the present disclosure, unless otherwise specified, the terms used to denote the orientations, such as "upper", "lower", "left", "right", "top" and "bottom", usually refer to the orientations as indicated in the figures; and "inside" and "outside" usually refer to inside and outside with respect to the outlines of the components.

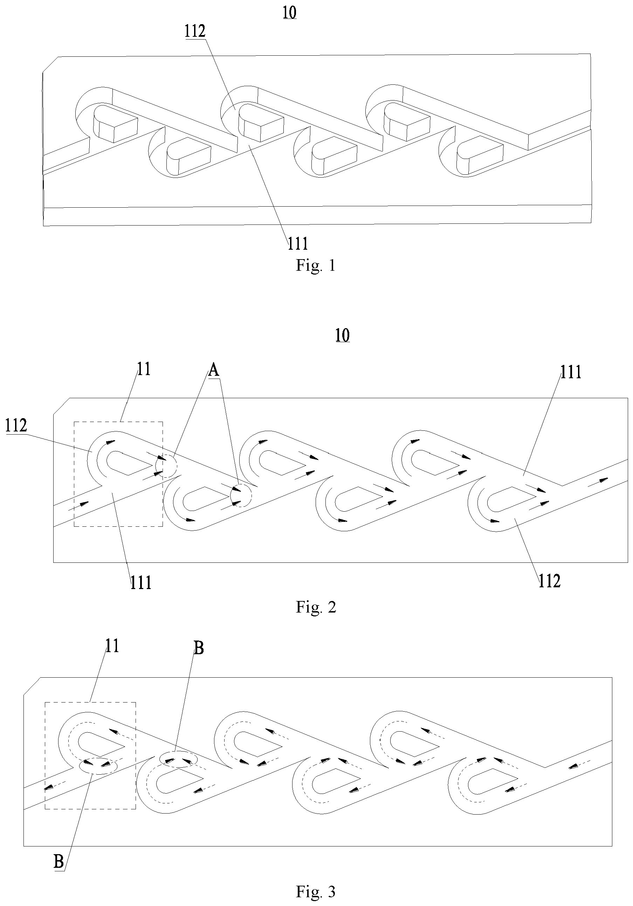

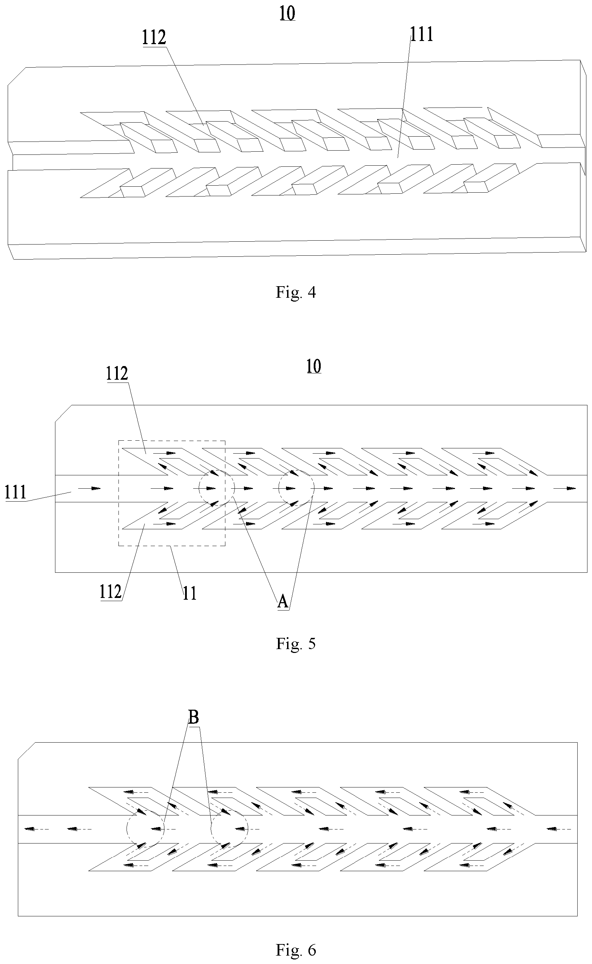

In a first aspect, the present disclosure provides an air inlet structure 10, which comprises one or more air inlet units 11, each air inlet unit 11 comprises a main air path 111 and at least one branch air path 112 configured to bend laterally and extend from the upstream of the main air path 111 to the downstream of the main air path 111, wherein at the position where the branch air path 112 is in communication with the upstream of the main air path 111 (see position B in Fig. 3 or 6), an included angle between the direction of an airflow entering the branch air path 112 and the direction of an airflow in the main air path 111 is an obtuse angle; at the position where the branch air path 112 is in communication with the downstream of the main air path 111 (see position A in Fig. 2 or 5), an included angle between the direction of an airflow from the branch air path 112 and the direction of an airflow in the main air path 111 is an acute angle;

In the above description, it can be understood that the two ends of the air inlet structure 10 in the airflow direction are an air inlet end and an air outlet end respectively. The upstream and downstream of the main air path 111 are also defined based on the airflow direction.

With the above technical scheme, when the air inlet structure 10 in the present disclosure is in use, the airflow entering through the air inlet end (see the left end in Fig. 2 or 5) of the air inlet structure 10 can flow towards the downstream along the main air path 111 and the branch air path 112 respectively (see the solid arrow in Fig. 2 or 5); the airflow in the main air path 111 and the airflow in the branch air path 112 are converged at a position where the branch air path 112 is in communication with the main air path 111 at the downstream of the main air path 111 to form an airflow convergence point A (see Fig. 2 or 5), so that the air flow speed after the airflows are converged is increased, thereby the flow of air is prompted. The noise that enters reversely through the air outlet end (see the right end in Fig. 3 or 6) of the air inlet structure 10 can be propagated reversely along the main air path 111 and the branch air path 112 respectively (indicated by the dotted arrow in Fig. 3 or 6); the noise propagated along the main air path 111 and the noise propagated along the branch air path 112 are converged at a position where the branch air path 112 is in communication with the main air path 111 at the upstream of the main air path 111 to form a noise convergence point B; at the noise convergence point B, the noise propagation direction in the branch air path 112 is opposite or approximately opposite to the noise propagation direction in the main air path 111, and the noises cancel each other out, thereby an effect of noise reduction is achieved. When the air inlet structure 10 in the present disclosure is applied to the ventilation treatment device, noise transmitted from an air intake passage of a blower can be effectively reduced, and the air inlet structure 10 is convenient to process, simple in structure, and convenient to apply.

According to a preferred embodiment of the present disclosure, the air inlet unit 11 comprises a plurality of branch air paths 112, which are connected in parallel with each other to the main air path 111 (see Fig. 4).

In the present disclosure, the air inlet structure 10 may comprise one or more air inlet units 11. According to a preferred embodiment of the present disclosure, the air inlet structure 10 comprises a plurality of air inlet units 11, which are in communication with each other sequentially via the main air paths 111. The more the air inlet units 11 are, the more the air flow speed is increased, which is more conducive to rapid flow of the airflow, and the noise reduction effect is more significant.

In the present disclosure, the main air path 111 may extend in a variety of forms, such as linear extension or curved extension. For example, in the embodiment shown in Figs. 1 and 4, the main air path 111 is in a linear shape. In addition, the branch air path 112 may also extend in a variety of forms, as long as it extends beside the main air path 111 from the upstream to the downstream of the main air path 111. For example, in the embodiment shown in Figs. 1-3, the branch air path 112 is in an arc shape. For example, in the embodiment shown in Figs. 4-6, the branch air path 112 is in a trilateral frame shape.

Specifically, please see the air inlet structure 10 shown in Figs. 1-3. The air inlet structure 10 comprises a plurality of air inlet units 11, which are in communication with each other sequentially via main air paths 111; the main air paths 111 of adjacent air inlet units 11 are arranged at an included angle; each air inlet unit 11 comprises one main air path 111 and one branch air path 112, wherein the main air path 111 is in a linear shape, and the branch air path 112 is in an arc shape.

Please see the air inlet structure 10 shown in Figs. 4-6. The air inlet structure 10 comprises a plurality of air inlet units 11, which are in communication with each other sequentially via main air paths 111; the plurality of main air paths 111 are arranged in a straight line; each air inlet unit 11 comprises one main air path 111 and two branch air paths 112, wherein the main air path 111 is in a linear shape, and each branch air path 112 is in a trilateral frame shape.

It may be noted that the air inlet structure 10 in the present disclosure can be used for noise reduction in scenarios where the air flow direction in the air path is opposite to the noise propagation direction.

In a second aspect, the present disclosure provides an air inlet component, which comprises the air inlet structure 10 described above.

The air inlet component may be any component used for bearing the air inlet structure 10, such as an upper housing in a noise reduction box to be described below.

The air inlet component may have an air inlet and an air outlet, wherein the air inlet is in communication with an air inlet end of the air inlet structure 10, and the air outlet is in communication with an air outlet end of the air inlet structure 10.

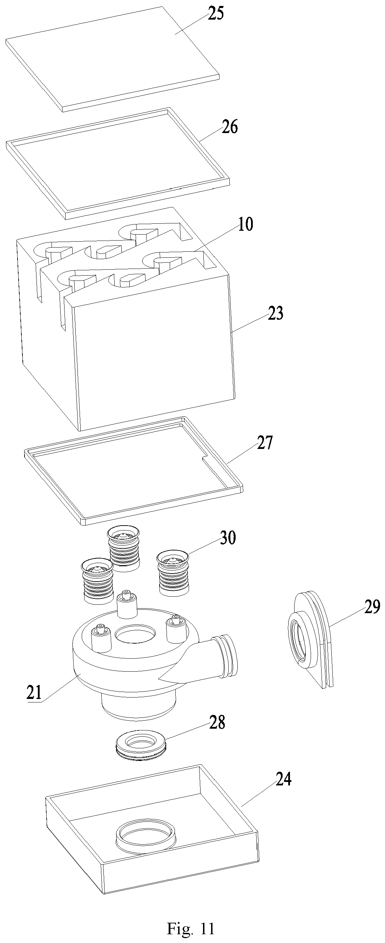

The air inlet component may comprise one or more air inlet structures 10. In the case that the air inlet component comprises a plurality of air inlet structures 10, the plurality of air inlet structures 10 may be arranged side by side, and the air inlet component may have one air inlet and one air outlet, or may have a plurality of air inlets and a plurality of air outlets, corresponding to the number of the air inlet structures 10. For example, the upper housing 23 shown in Fig. 11 comprises two air inlet structures 10 arranged side by side.

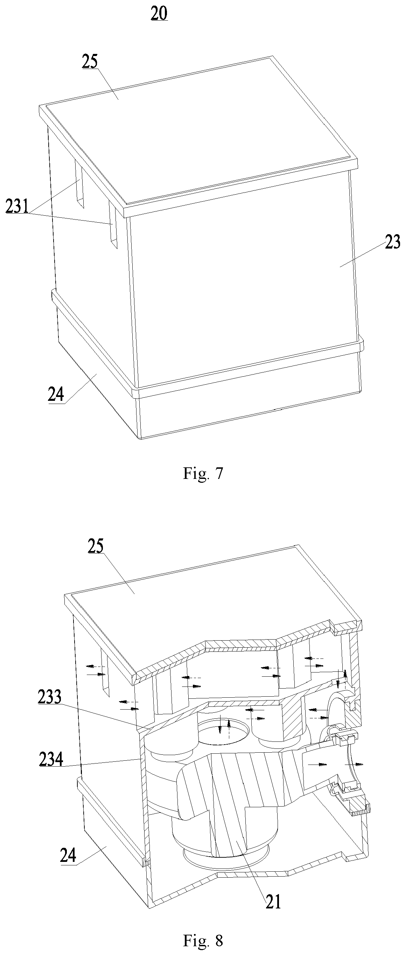

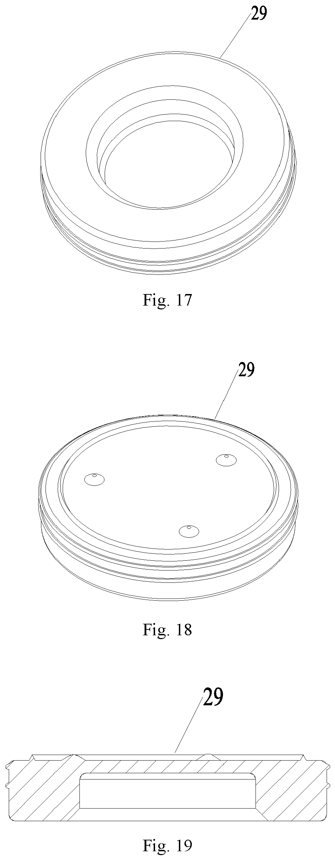

In a third aspect, the present disclosure provides a noise reduction box 20. As shown in Figs. 7-19, the noise reduction box 20 comprises a housing and a blower 21, wherein the housing has a mounting cavity 22, and an air inlet 231 and an air outlet 232 that are in communication with the mounting cavity 22; the blower 21 is mounted in the mounting cavity 22, an air intake port 211 of the blower 21 is in communication with the air inlet 231, an air discharge port 212 of the blower 21 is in communication with the air outlet 232, and a communication path between the air inlet 231 and the air intake port 211 is provided with the air inlet structure 10.

In the above description, it can be understood that the air inlet end of the air inlet structure 10 is in communication with the air inlet 231, and the air outlet end of the air inlet structure 10 is in communication with the air intake port 211. In use, air can enter the air inlet structure 10 through the air inlet 231 of the housing and the air inlet end of the air inlet structure 10 sequentially, flow to the air outlet end of the air inlet structure 10 after the air flow speed is increased, then enter the blower 21 through the air intake port 211 of the blower 21, and finally flow out of the noise reduction box 20 through the air discharge port 212 of the blower 21 and the air outlet 232 of the housing. After the noise generated by the high-speed operation of the blower 21 is propagated through the air intake port 211, it will enter the air inlet structure 10 through the air outlet end of the air inlet structure 10 for noise reduction.

In order to facilitate the installation and maintenance of the components, the housing may comprise an upper housing 23 and a lower housing 24 assembled below the upper housing 23, the mounting cavity 22 is defined by the upper housing 23 and the lower housing 24 together, and the air inlet 231 and the air inlet structure 10 can be arranged on the upper housing 23.

Specifically, please see Figs. 8-10 and 12. The upper housing 23 may comprise a top wall 233 and a peripheral wall 234 extending downward from the periphery of the top wall 233; the air inlet 231 and the air inlet structure 10 are arranged on the top wall 233, and the top wall 233 is provided with a communication port 235 for communication between the air outlet end of the air inlet structure 10 and the mounting cavity 22. The air outlet 232 may be formed on the peripheral wall 234.

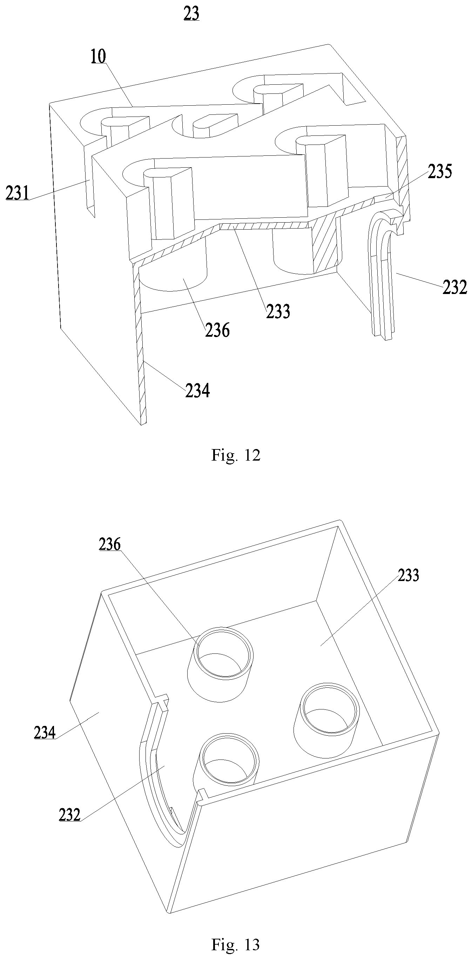

It may be noted: in the case that a plurality of air inlet structures 10 are provided, there may be a plurality of air inlets 231 and a plurality of communication ports 235, for example, as shown in Figs. 11 and 12.

In order to facilitate the processing of the air inlet structure 10, as shown in Fig. 12, the air inlet structure 10 is preferably arranged on the top surface of the top wall 233 in a recessed form. In that case, the housing may further comprise a top cover plate 25, which covers above the top wall 233.

As shown in Figs. 7-11, in order to improve the sealing performance when the top cover plate 25 and the upper housing 23 are assembled, an upper seal 26 may be provided between the top cover plate 25 and the upper housing 23. In order to improve the sealing performance when the upper housing 23 and the lower housing 24 are assembled, a lower seal 27 may be provided between the upper housing 23 and the lower housing 24.

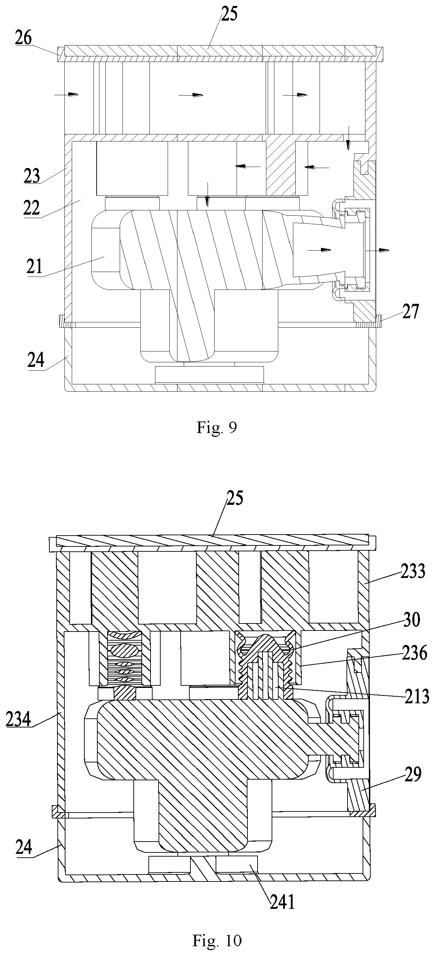

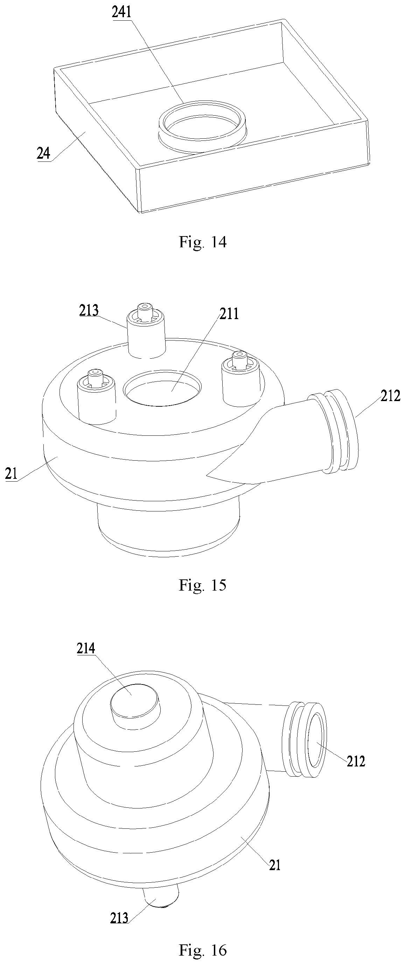

In addition, in order to reduce the vibration noise of the blower and position the blower 21 in the mounting cavity 22, the noise reduction box 20 may further comprise a positioning ring 28, a seal ring 29 and shock-absorbing legs 30. As shown in Figs. 10 and 15, blower legs 213 are provided on the top of the blower, and the shock-absorbing legs 30 are sleeved on the blower legs 213 and inserted into mounting cylinders 236 extending downward from the top wall 233. As shown in Figs. 10 and 16, a positioning column 214 is provided on the top of the blower, and the positioning ring 28 is sleeved on the positioning column 214 and loaded into a positioning cylinder 241 extending upward from the lower housing 24. As shown in Fig. 10, the seal ring 29 may be mounted at the air discharge port 212 of the blower and the air outlet 232 of the housing.

It may be noted: to facilitate understanding and to illustrate the structure better, the orientational words "upper", "lower", "top" and "bottom" used in the above description of the noise reduction box refer to the orientations shown in the corresponding drawings. When the noise reduction box is installed and in use, it can be placed upside down.

In a fourth aspect, the present disclosure provides a ventilation treatment device, which comprises the noise reduction box 20 described above.

The ventilation treatment device may further include a main unit, a ventilation pipeline and a patient interface unit, wherein the main unit has a cavity, and a main unit air inlet and a main unit air outlet that are in communication with the cavity; the noise reduction box 20 may be arranged in the cavity, the air inlet 231 of the noise reduction box 20 may be in communication with the main unit air inlet, and the air outlet 232 of the noise reduction box 20 may be in communication with the main unit air outlet. One end of the ventilation pipeline is connected to the main unit air outlet, and the other end of the ventilation pipeline is connected to the patient interface unit, so as to transfer the respiratory air produced by the main unit to the patient interface unit for inhalation by the patient.

The patient interface unit may be a device for communication with the mouth and/or nose of the patient, such as a respiratory mask, a nasal oxygen cannula, or the like.

The ventilation treatment device may be a ventilator or an oxygen therapy apparatus, or the like.

While some preferred embodiments of the present disclosure are described above with reference to the accompanying drawings, the present disclosure is not limited to the details in those embodiments. Those skilled in the art can make various simple modifications and variations to the technical scheme of the present disclosure, without departing from the technical concept of the present disclosure. However, all these simple modifications and variations shall be deemed as falling in the scope of protection of the present disclosure.

In addition, it may be noted that the specific technical features described in the above embodiments may be combined in any appropriate form, provided that there is no conflict among them. To avoid unnecessary repetition, various possible combinations are not described specifically in the present disclosure.

Moreover, different embodiments of the present disclosure may also be combined freely as required, as long as the combinations don't deviate from the ideal of the present disclosure. However, such combinations shall also be deemed as being disclosed in the present disclosure.

## Claims

1. An air inlet structure (10), comprising one or more air inlet units (11), each air inlet unit (11) comprises a main air path (111) and at least one branch air path (112) configured to laterally bend and extend from the upstream of the main air path (111) to the downstream of the main air path (111), wherein at the position where the branch air path (112) is in communication with the upstream of the main air path (111), an included angle between the direction of an airflow entering the branch air path (112) and the direction of an airflow in the main air path (111) is an obtuse angle; and at the position where the branch air path (112) is in communication with the downstream of the main air path (111), an included angle between the direction of an airflow from the branch air path (112) and the direction of an airflow in the main air path (111) is an acute angle.

2. The air inlet structure of claim 1, wherein
each air inlet unit (11) comprises a plurality of branch air paths (112) connected in parallel with each other to the main air path (111); and/or
the air inlet structure (10) comprises a plurality of air inlet units (11) in communication with each other sequentially via the main air paths (111).

3. The air inlet structure of claim 1, wherein the main air path (111) is in a linear shape, and/or each branch air path (112) is in an arc shape or trilateral frame shape.

4. The air inlet structure of claim 1, wherein
the main air path (111) is in a linear shape, each branch air path (112) is in an arc shape, and the air inlet structure (10) comprises a plurality of air inlet units (11) in communication with each other sequentially via the main air paths (111), and the main air paths (111) of adjacent air inlet units (11) are arranged at an included angle with respect to each other; or
the main air path (111) is in a linear shape, each branch air path (112) is in a trilateral frame shape, and the air inlet structure (10) comprises a plurality of air inlet units (11) in communication with each other sequentially via the main air paths (111), and the plurality of main air paths (111) are arranged in a straight line.

5. An air inlet component, comprising the air inlet structure (10) of any of claims 1-4.

6. The air inlet component of claim 5, wherein
the air inlet component has an air inlet and an air outlet, wherein the air inlet is in communication with an air inlet end of the air inlet structure (10), and the air outlet is in communication with an air outlet end of the air inlet structure (10); and/or
the air inlet component comprises a plurality of air inlet structures (10) arranged side by side.

7. A noise reduction box (20), comprising a housing and a blower (21), wherein the housing has a mounting cavity (22), and an air inlet (231) and an air outlet (232) that are in communication with the mounting cavity (22); the blower (21) is mounted in the mounting cavity (22), an air intake port (211) of the blower (21) is in communication with the air inlet (231), an air discharge port (212) of the blower (21) is in communication with the air outlet (232); and a communication path between the air inlet (231) and the air intake port (211) is provided with the air inlet structure (10) of any of claims 1-4.

8. The noise reduction box of claim 7, wherein the housing comprises an upper housing (23) and a lower housing (24) assembled below the upper housing (23), the mounting cavity (22) is defined by the upper housing (23) and the lower housing (24) together, and the air inlet structure (10) is arranged on the upper housing (23).

9. The noise reduction box of claim 8, wherein the upper housing (23) comprises a top wall (233) and a peripheral wall (234) extending downward from the periphery of the top wall (233), the air inlet (231) and the air inlet structure (10) are arranged on the top wall (233), and the top wall (233) is provided with a communication port (235) for communication between an air outlet end of the air inlet structure (10) and the mounting cavity (22).

10. The noise reduction box of claim 9, wherein the air inlet structure (10) is arranged on a top surface of the top wall (233) in a recessed form, and the housing further comprises an upper cover plate (25), which covers above the top wall (233).

11. A ventilation treatment device, comprising the noise reduction box (20) of any of claims 7-10.
